(19) Europäisches Patentamt
European Patent Office
Office européen des brevets

(11) Publication number: **0 086 757**
**A1**

# (12) EUROPEAN PATENT APPLICATION

(21) Application number: **83830030.9**

(22) Date of filing: **09.02.83**

(51) Int. Cl.³: **C 07 C 153/09**, A 61 K 31/265
// C07C103/48

(30) Priority: **12.02.82 IT 1965082**

(43) Date of publication of application: **24.08.83**
**Bulletin 83/34**

(84) Designated Contracting States: **AT BE CH DE FR GB LI LU NL SE**

(71) Applicant: **Farmatis S.r.l., Corso Europa n. 5, I-20100 Milano 1 (IT)**

(72) Inventor: **Scalesciani, Juan Boris Aldo, Calle J.B. Alberdi, Buenos Aires (AR)**

(74) Representative: **Gervasi, Gemma et al, Studio Brevetti e Marchi NOTARBARTOLO & GERVASI 33, Viale Bianca Maria, I-20122 Milano (IT)**

(54) **Derivatives of alpha or beta-mercaptopropionylamidoacetic acids, processes for their preparation and the therapeutic compositions which include them as active principles.**

(57) New derivatives of alpha or beta-mercaptopropionylamidoacetic acids have been prepared, having the following formula:

$$R_2CO\text{-}S\text{-}(\underset{R_1}{CH})_n\text{-}CO\text{-}NH\text{-}CH_2\text{-}COOH \qquad (I)$$

in which $R_1$ = H, $CH_3$; $R_2$ = alkylic radical, linear or with 8-20 carbon-atom branches; n = 1 or 2 and their salts.

The new compounds are very good mucolytics, hepta-protectors and antihypertensives.

EP 0 086 757 A1

0086757



"Derivatives of alpha or beta-mercaptopropionylamidoacetic acids, processes for their preparation and the therapeutic compositions which include them as active principles"

This invention concerns a new class of alpha or beta--mercaptopropionylamidoacetic acid derivatives, the process for preparing them and their use as active principles in compositions which have mucolytic, hepato--protective and antihypertensive action.
This invention concerns precisely the compounds included in the following general formula:

$$R_2\text{-CO-S-}\underset{\substack{| \\ R_1}}{(\text{CH})_n}\text{-CO-NH-CH}_2\text{-COOH} \qquad (I)$$

in which $R_1$ = H or $CH_3$;
n = 1 or 2;
$R_2$= alkylic radical, linear or with 8-20 carbon-atom branches, and their salts.
The alpha-mercaptopropionylamidoacetic acid derivatives of this invention have this general formula:

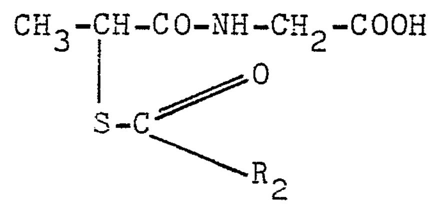

(II)

in which $R_2$ is as previously defined.
The beta-mercaptopropionylamidoacetic acid derivatives of this invention have this general formula :

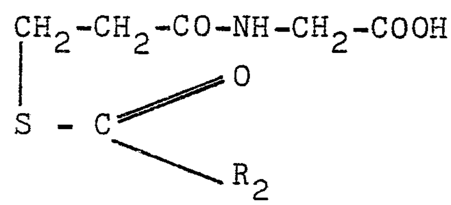

(III)

In which $R_2$ is as previously defined.
The formula (I) compounds are prepared according to this invention, by reacting glycine with an halogenide of alpha-bromo acid or beta-bromo-propionic acid.
The bromo-propionamidoacetic acid thus obtained is then made to react with thioacids having the following general formula:

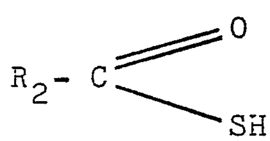

(IV)

in which $R_2$ = alkylic radical, linear or with 8-20 carbon atom branches.
The IV compounds can be prepared in accordance with Y. Hirabayashi Bull. Chem. Soc. Japan, 39, 2216 - (1966).
The new process is illustrated by the reactions shown below:

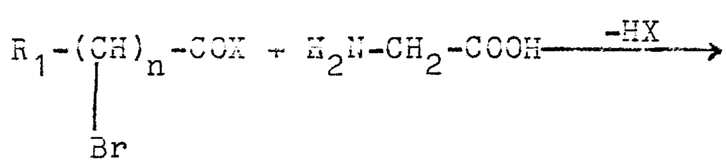

$$\longrightarrow R_1\text{-}\underset{\displaystyle Br}{(CH)_n}\text{-}CO\text{-}NH\text{-}CH_2\text{-}COOH$$

X = Cl. Br.

2)

$$R_1\text{-}CH)_n\text{-}CO\text{-}NH\text{-}CH_2\text{-}COOH + R_2\text{-}C\begin{matrix}O\\ \\ SH\end{matrix} \xrightarrow{-HBr}$$

$$\longrightarrow R_1\text{-}\underset{\displaystyle S\text{-}COR_2}{(CH)_n}\text{-}CO\text{-}NH\text{-}CH_2\text{-}COOH \qquad (I)$$

in which $R_1$, $R_2$, X and n are as previously defined.

The (I) compounds thus obtained are eventually saltified.

Phase (1) of the above process is carried out in an aqueous solution in the presence of mineral bases.

The reaction is carried out under energetic agitation, cooling in a water-ice bath to obtain a preferred temperature of not over 10°C.

The bromo-propionamidoacetic acid is prepared by concentrating the solution to dryness and crystallizing the residue with the use of an organic solvent.

The products obtained in phase (1) are made to react with thioacids having the general formula (IV) in dipolar, aprotonic, water-mixable solvents, such as DMF and DMSO, in the presence of an organic base, in a quantity which is at least chemically sufficient to block the HBr which is formed.

The reaction is made to take place under agitation and at room temperature.

0086757

The final products (I), which are obtained at almost quantitative results, are acidified and separated by diluting with water.

The following examples are given so as to facilitate the reproduction of the process involved in this invention. The examples are chosen so as to be practical and illustrative without inferring any limitations.

EXAMPLE 1

Alpha-bromopropionylamidoacetic acid

To a solution of 75 g of glycine in 500 ml of 2N, aqueous sodium hydrate, cooled in an ice bath, 550 ml of 2N, aqueous sodium hydrate and 180 g of alpha-bromopropionylchloride are alternately added, in portions, while agitating vigorously.

Agitation is continued for four hours at room temperature, after which the reaction mixture is extracted using ethylic ester. After acidifying with conc. HCL, extraction is performed three times on the aqueous solution, using ethyl acetate.

The organic material extracted is allowed to dry on sodium sulfate and dry-concentrated. The residue is crystallized with chloroform. 190 g of alpha-bromopropionylamideacetic are obtained having a m.p. of 104°C.

EXAMPLE 2

Beta-bromopropionylamidoacetic acid

Beta-bromopropionylamidoacetic acid is obtained in the same

way as was done in Example 1, by using beta-bromopropionylchloride instead of alpha-bromopropionylchloride.

EXAMPLE 3

Alpha-myristoylmercaptopropionylamidoacetic acid

To a solution of 21.0 g of alpha-bromopropionylglycine in 50 ml of dimethylformamide and 30 ml of triethylamide, 24.4 g (0.10 moles) of tetradecanthioic acid - dissolved in 50 ml of dimethylformamide - are added while agitating. The reaction is allowed to take place at room temperature for 4 hours under constant agitation. The mixture is then diluted with 500 ml of water and acidified with conc. HCL up to a pH value of $\simeq 2$. The solid material which forms is filtered out, washed copiously with water, dried under a vacuum and crystallized with ethyl acetate.

33.6 g of alpha-myristoylmercaptopropionylamidoacetic acid are thus obtained having a m.p. of 97°C.

All the products were obtained in an analogous manner. The essential characteristics of these products are shown in Tables 1 and 2.

The obtained acids can be transformed into the corresponding salts by treating with dipolar, aprotonic solvents with alkaline alcoholates (n.d.t. H of OH group replaced by monovalent metal, in general) or with organic bases, followed by precipitation with water.

TABLE 1

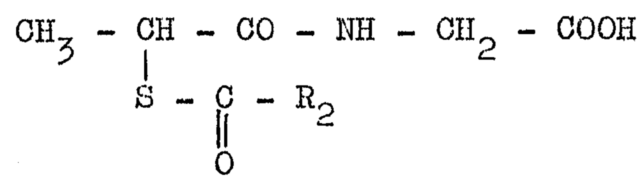

| $R_2$ | Formula | M.W | M.P. | Elemental Analysis | C | H | N | S |
|---|---|---|---|---|---|---|---|---|
| Lauroyl | $C_{17}H_{31}NO_4S$ | 345.6 | 93° | Calculated | 59.1 | 9.0 | 4.1 | 9.3 |
| | | | | Found | 59.0 | 8.9 | 4.1 | 9.2 |
| Myristoyl | $C_{19}H_{35}NO_4S$ | 373.6 | 97° | Calculated | 61.1 | 9.4 | 3.8 | 8.6 |
| | | | | Found | 61.1 | 9.4 | 3.8 | 8.6 |
| Palmitoyl | $C_{21}H_{39}NO_4S$ | 401.6 | 99° | Calculated | 62.8 | 9.8 | 3.5 | 8.0 |
| | | | | Found | 62.5 | 9.6 | 3.5 | 7.9 |

TABLE 2

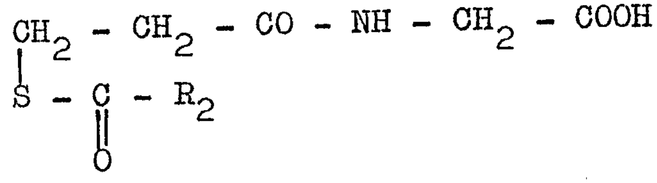

| $R_2$ | Formula | M.W. | M.P. | | Elemental Analysis C | H | N | S |
|---|---|---|---|---|---|---|---|---|
| Lauroyl | $C_{17}H_{31}NO_4S$ | 345.6 | 131° | Calculated<br>Found | 59.1<br>59.0 | 9.0<br>8.9 | 4.1<br>4.1 | 9.3<br>9.4 |
| Myristoyl | $C_{19}H_{35}NO_4S$ | 373.6 | 130° | Calculated<br>Found | 61.1<br>61.0 | 9.4<br>9.5 | 3.8<br>3.8 | 8.6<br>8.7 |
| Palmitoyl | $C_{21}H_{39}NO_4S$ | 401.6 | 129° | Calculated<br>Found | 62.8<br>62.3 | 9.8<br>9.7 | 3.5<br>3.5 | 8.0<br>8.1 |

As previously mentioned, the new products of this invention have shown intense 1) mucolytic-mucoregulating activity, 2) hepatotrophic-disintoxicating activity and 3) antihypertensive activity.

The following information and significant data are given to permit proper evaluation of the new products, with the understanding that what is stated holds true for all the products considered which, from all the pharmacological and clinical test results, have proved to be substantially equivalent.

1. Mucolytic-mucoregolating activity

It is known that the liquefying of excreta by chemical means constitutes a fundamental therapeutic objective in the modern therapy regarding obstructive, chronic bronchopneumonopathics. Since the bronchial secretion generally consists of purulent exudates and mucous - the exudates being DNA-rich and the mucous mucoprotein-rich, the chemical products used up to now as liquefiers have been enzymes, surfactants and sulfurated compounds.

The enzymes and surfactants are not, however, consistent in their effectiveness and are not well tolerated, giving rise to allergies, bronchial asthma attacks and irritation of the mucous membranes. The sulfurated compounds are the most interesting mucolytics today, especially L-cysteine and some of its derivatives. Because of the instability of natural aminoacids in solution, the offensive level of the odor and the short activity period due to rapid metabolizing, the derivatives are preferred over the L-cysteine.

The common characteristic of all these sulfurated compounds

is the presence of the -SH groups, which are essential for providing the mucolytic action. In fact, the protection of this group by acetylation or its oxidation to disulfide makes the mucolytic activity disappear (Sheffner A.L., The reduction "in vitro" in viscosity of mucoprotein solutions by a new mucolytic agent, N-acetyl-L-cysteine. Ann. N.Y. Acad. Sci., 106, 298 - 1963).
The mechanism of the mucolytic action for the L-cysteine derivatives - both "in vitro" and for local application by means of instillation or aerosol therapy - seems to derive from a thiol-disulfide interaction with a breaking of the intramolecular cysteinic disulfide bridges of the mucoprotein with consequent reduction of the mucoprotein. When these pharmaceutical products are administered orally or parenterally, there seems to be, instead, a regulating effect on the cellular metabolism and, in particular, on the enzymatic activity, as well as on the oxidizing-reducer processes of the respiratory chain and on the permeability of the membranes at the bronchial level where the thiolic compounds tend to accumulate.
These latter properties are more mucoregulatory than mucolytic and are accentuated in carboxymethylated cysteine at the thiolic group, which is almost without mucolytic activity "in vitro" but active "in vivo" and better tolerated than the derivatives of cysteine with free thiol. During aerosol therapy, in fact, these derivatives free small quantities of sulfurated hydrogen which have an intense local irritating action with consequent manifestations of broncospasm and hypersecretion of tracheobronchial fluid, giving rise to a feeling of suffocation, nausea and vomiting.

They are also often irritating to the gastric mucosa, when administered orally or parenterally, with eructation having a nauseating taste and odor of mercaptan. (Bernstein I.L., Ausdenmoore R.W., Iatrogenic bronchospasm occurring during clinical trials of a new mucolytic agent, acetylcysteine. Dis. Chest. 46, 469 - 1964).

S-carboxymethyl-cysteine, instead, is suitable for oral administration and has no irritative action because it does not oxidize when exposed to the air. It does not act before being converted to a thiolic compound followed by the thiol-disulfide interaction, but modifies the composition of the bronchial mucosa, diminishing the fucus-rich, neutral glycopeptides (hydrophobics) and augmenting the hydrophile sialoglycopeptides.

The new compounds with the thiolic group protected by superior fatty acid esterification, object of this invention, quite unexpectedly displayed tolerability and mucolytic and mucoregulatory activity characteristics that were even superior to those shown by S-carboxymethyl-cysteine.

More precisely, the advantages determined for these new compounds with respect to the thiolic derivatives with known techniques can be stated as follows:

1) Gradual activity with reduced local irritation due to the absence of free thiolic groups and to the impossibility of the forming of rapidly-biodegradable cysteine (essential difference from S-carbosymethylcysteine).

2) Greater absorbability, due to the superior liposolubility and transmucosal penetrability, with high tolerability at the bronchial mucosa level and alveolar epithelium of the bronchi.

BAD ORIGINAL

due to the affinity which exists - particularly with the palmitic esters - with the pulmonary surfactant (dipalmitoyl-lecithin) which is responsible for maintaining alveolar stability and pulmonary expandability.

The new derivatives of mercaptopropionamidoacetic acid underwent "in vitro" tests based directly on viscosimetric variations of the bronchial excreta and/or gastric mucin of swine. The results shows almost complete lack of activity, as was to be expected since there were no free thiolic groups.

The "in vitro" tests, however, were performed on the excreta of patients with bronchial hypersecretion, administering the esters orally (30 and 50 mg/kg/die, respectively) for a period of one week, with the result that these new compounds proved effective in modifying the mucin constituents of the fibrillar reticulum. Analysis of the glycopeptides of the bronchial mucus - which were obtained by means of hydrolysis with papain (24 hours at 37° and pH 6.5) and then with pronase (24 hours at 37° and pH 7.3) and fractioned by electrophoresis on agarose at pH 8.6 followed by chromatographic analysis on column DEAE-Sephadex A/25 - in fact demonstrated an average increase of sialoglycopeptides (hydrophile) of almost 90% and a reduction of over 55% of the weakly acid glycopeptides and neutral mucin (hydrophobic) with consequent diminution of the viscosity. Comparative tests with S-carboxymethylcysteine at higher doses (70 mg/kg/die) showed an average increase in sialoglycopeptides that did not exceed 75% and a diminution of glycopeptides and mucin that did not exceed 45%.

Even "in vivo" the mucolytic and mucoregulating activity of

BAD ORIGINAL

0086757

the new compounds proved intense, in tests which evaluated the bronchial hypersecretion induced in rats with $SO_2$ after having administrated the esters orally at the rate of 0.5 g/kg/die for two weeks. The inhalation of 300 ppm of $SO_2$ for two hours per day, for a total period of 90 hours, produced bronchial obstruction in only 12% of the animals compared to the 80% produced in those not treated.

Under the same test conditions, using the same dose of 0.5 g/kg/die of S-carboxymethylcysteine, the per cent of animals not protected was 25%.

Histological and histochemical (P.A.S. coloration) tests, furthermore, showed that the bronchial mucosa of the rats treated with the new compounds had a considerable significant reduction (about 35% less) of the calyciform hyperplasia and the number of muciparous cells, with the almost complete persistence of the -SH groups in the cells of the bronchial mucosa otherwise reduced by about 70% by the $SO_2$. These results can be attributed to a cytotrophic-protective action.

Finally, with the rats which underwent $CCl_4$ nebulization treatment for 10 days (5 applications, 2 minutes per day), the contemporaneous oral administration of the esters of this invention, at doses of 0.5 g/kg/die, prevented intrabronchial desquamation and reactive hyperplasia of the mucosa to the extent of 60%. Under the same test conditions, with the same doses, S-carboxymethylcysteine produced prevention to an extent which did not exceed 40%.

These results also attest to the new compounds' capability of producing trophic-protective action.

The anti-inflammatory activity of the new compounds was also

BAD ORIGINAL

determined because of the importance of phlogosis in the bronchospasmic processes.

The anti-inflammatory activity was investigated on rats by administering the new products, orally, using an esophagal probe. One hour after administration, a subcutaneous injection of 0.05 ml of 1% carragenin was made in the right hind paw and three hours later a plethysmometric evaluation was made of the difference in volume between the two hind paws (WINTER C.A., RISLEY E.A., NUSS G.W., Carragenin-induced edema in hind paw of the rat as an assay for anti-inflammatory drugs. Proc. Soc. Exp. Biol. Med., 111, 544 - 1962).

The $DE_{50}$ of the new products proved to be about 1 g/kg while that of S-carboxymethylcysteine was not determinable. By way of comparison, we would say that the $DE_{50}$ of a classical antiphlogistic, such as acetylsalicylic acid, is 100 mg/kg.

The esters with which this invention is concerned, therefore, have an antiphlogistic capability which is able to inhibit plasmatic quinines which consequentially favors the reduction of bronchospasm in obstructive and hypersecretive bronchopneumonopathics.

The antibronchospastic activity of the new compounds, in fact, was evaluated by pretreating test animals with 2 g/kg of the new compounds, administered by way of an esophagal probe, and then submitting them to aerosol histaminic shock (histamine phosphate 1:80 at about 140 mm Hg). The time was noted between when the aerosol was given and the convulsions began. After two hours of pretreatment, times amounting to 70% of the approximately 120 seconds of the control group were obtained. Induction times proved even greater by 20% after

BAD ORIGINAL

48 hours. With S-carboxymethylcysteine there was no appreciable difference between the control and tested subjects. The new compounds, therefore, provide intense and persistent protection against bronchspasm induced experimentally with histamine, in strict correlation with the anti-inflammatory activity reported previously.

All the "in vivo" tests described above demonstrate that these new compounds have an intense mucoregulation activity and cytotrophic-protective activity on the bronchial mucosa; and also have anti-inflammatory and antibronchospasmic effectiveness which is superior to that of S-carboxymethylcysteine. Furthermore, they diminish hypersecretion and phlogosis, which is experimentally induced, thus reequilibrating the glycopeptide fractions of the fibrillar mucin and modifying the rheological quality of the pathological mucous which, after oral treatment, becomes less viscous without, however, becoming too fluid, as instead happens after treatment with thiolic compounds.

Preliminary clinical tests on chronic bronchitis cases, where the new compounds were administered orally (3 g) or rectally (0.9 g), these amounts daily for at least a week, showed a gradual reduction of viscosity of the excreta (an average of 70% for purulent expectoration and 50% for mucopurulent expectoration) 4-5 days after the beginning of treatment (evaluation was made with a rotary, cone and plate viscosimeter), and an increase in vital capacity of 20%, an increase of 15% in VEMS and a residual volume reduction of about 20%.

The new compounds can also be administered using aerosol, dispersed from 5-10% in a 0.15 M sodium chloride solution. The

BAD ORIGINAL

aerosol action is produced by an ultrasonic unit at 37°C. The particles produced are submicronic (0.1 - 0.6 micron), very stable, and are able to assure the penetration of other pharmaceutical products, such as chemio-antibiotics, into the pumonary alveoli.

The administration by aerosol did not reveal any quinic action of the kallikrein on the bronchial tone. In other words, no bronchospasmic manifestations were noted (evaluated by means of plethysmograph with broncholabile patients). This test also demonstrates the good level of tolerability of these new compounds in comparison to the free thiolic compounds.

2. Hepatotrophic-disintoxicating activity

After the treatment described in the preceding paragraph where $CCl_4$ was used - a treatment which notoriously provokes hepatic alterations which are similar to those produced by viral hepatitis - liver examinations were made. It was unexpectedly found that the new products had produced a very strong antitoxic, hepato-protective action.

In fact, it was seen that, in these rats, the weight of the liver represented only 4% of the total body weight. When the liver checks were made, the weight of the liver constituted about 6% of the total body weight, which shows that the new compounds impede the increase of weight of the hepatic parenchyma induced by the lesioning action of the $CCl_4$ on the liver. Macroscopic examination confirmed these results. The livers of the animals in the control group were greatly increased in size, pale in color, with increased consistency but fragile and with a microgranulous surface. The treated animals presented a much better picture, being practically

BAD ORIGINAL

normal. The histological examination showed the control animals as having the classical alterations induced by the hepatotoxin (cytoplasmatic vacuolation, hepatocyte volume at least tripled, albuminoid degeneration and notably proliferated, fatty connective tissue with the formation of pseudolobes). In the treated animals, it was evident that the damage to the liver had been normalized (hepatocytes practically normal in size, with microvacuolation - often with two nuclei - demonstrating an active repair process, and slight connective-tissue proliferation.

Finally, while the livers of the control animals had a proteinic reduction of about 25%, those of the treated animals had a reduction of about 10%. Likewise, while there was an increase of 75% in the lipids content with the control group, it did not exceed 21% in the treated animals.

It is quite obvious that the hepatoprotective action demonstrated by the new compounds is due to its capability of inhibiting lipoperoxidation.

Assuming that the demolition of p-oxyphenylpyruvic acid is valid as a measure of the oxidative capacity of the hepatic microsomes, it is a significant fact that the increase in its urinary elimination from 10 to 20% of the dose administered (35 mg/kg per os at the 10th day of intoxication in the same rats which were exposed to $CCl_4$ inhalation) is still not greater than 13% of the animals treated with the new compounds.

These surprizing results with $CCl_4$ intoxication - which bring about modifications similar to those clinically observable in viral hepatitis cases - led to further hepatopathic experiments on rats using thioacetamide, which produces death in a way similar to that resulting from cirrhosis. The thioacetamide was given orally in doses of 185 mg/kg/die, while the new compounds were given orally in the same doses as in the previous tests: 0.5 g/kg/die. The contemporaneous treatment with the new compounds extended the survival period from 74 to 109 days, which amounts to about 47%.

A third experimental hepatopathic condition, characterized by hepatic necrosi and steatitis, was induced in rats by means of a single i.p. dose of etionine (0.4 g/kg), which normally causes the hepatic triglycerides to increase from 1 mg/g of liver weight to 4.5 mg/g, an increase of more than 400%. Pretreatment with the new compounds, giving oral doses of 0.5 g/kg/die, kept the increase to less than 200%.

Finally, with the hepatic steatitis cases - the rats being given the usual 0.5 g/kg/die does of the new compounds, orally, and kept on a Handler steatogenous diet - after 28 days the retarding of the clearance of the BSF (bronchosufonphthalene), which is an index of how much the liver function has been compromised, was observed to be 23% less than that of the animals which were only given steatogenous diet. After 30 minutes from the BSF e.v. injection, the hematic concentrations averaged 5.6% versus 7.3% of the control group.

All the above tests prove that the new compounds exert a definite hepatrophic-disintoxicating action which is, in a general sense, aspecific and polyvalent.

Clinically, preliminary tests were performed with the new compounds on a limited and differentiated group of test animals having, for the most part, acute hepatitis, which was chronic

and persistent and due to various causes, aggressive chronic hepatitis cases, compensated cirrhosis cases, all being given 3 g orally or 0.9 g rectally of the new compounds. The results were that, in addition to an improvement of the clinical symptomatology (asthenia, anorexia, nausea, dispepsia, epigastric heaviness and hepatomegaly), there was a decided recovery of the hematochemical cytolysis indexes, of the hepatocytic function and of the intrahepatic cholostasis, with very good local and system tolerability.

3. Antihypertensive activity

The antihypertensive activity of the new products was checked with induced hypertension using angiotensin I.

When rabbits with normal pressure were administered 0.5 g/kg/die of the new compounds for 10 days and then injected endovenously with 310 mg/kg of angiotensin I, their average arterial pressure went up not more than 5%. The non-pretreated animals had, instead, a pressure increase of 20%.

The preliminary clinical tests performed with the new compounds, administering 3 g orally and 0.9 g rectally per day for a period of from two to three weeks on subjects affected with essential arterial hypertension, demonstrated the new compounds' ability to reduce the arterial pressure independently of the basic values of the plasmatic renin activity. This reduction ability was enough to lead us to presume that the new compounds act with alternate mechanisms which block the production of angiotensin II.

Also in this case, even with the long treatment, a very unexpected and perfect tolerability was observed. As is known, thiolic products are often responsible for the reduction and

alteration of the gustatory sensitivity, as well as cutaneous itching and rash, when the therapy is protracted.

4. Toxicology

The new compounds have proved to have an extremely low level of acute toxicity. In fact, while S-carboxymethylcysteine has a $DL_{50}$ of 5.18 g/kg, the new thiolic esters' $DL_{50}$ cannot be determined, i.p., since more than 5 g/kg cannot be administered; but, orally, the $DL_{50}$ is above 8 g/kg, as with the S-carboymethylcysteine. The subacute, chronic and fetal toxicity was evaluated by mixing the esters with the food.

The subacute toxicity in rats was tested for 12 weeks; the chronic toxicity was tested in rats and dogs for six months (at 0.1 to 0.9 g/kg/die); the fetal toxicity was tested in rats (0.3 to 0.9 g/kg/die from the 6th to 16th day of gravidity) and in rabbits (0.1 to 0.3 g/kg/die from the 6th to 18th day of gravidity). All tests demonstrated very good tolerability to the new compounds.

In fact, no significant variance was observed in body weight, in the macro and microscopic aspects of the main organs, in the hemochromocytometric examinations, in the hepatic functional tests (bilirubin, SGOT, SGPT, alkaline phosphates), nor as regards uricemia, cholesterolemia, glycemia, azotemia and the standard urine tests. The treatment, furthermore, had no negative effects on the mothers, nor on the characteristics of the offspring as regards the number born, fetal losses and weights of offspring and fetuses, nor as regards embryonic and fetal development, on the basis of the number of malformations, variations or anomalies involving the skeleton or viscera.

BAD ORIGINAL

In order to evaluate the tolerability at the lung level, for the same rats undergoing the chronic toxicity test, a determination of the influence made at the surfactant level was done at the termination of the treatment. A phospholipids analysis was made, obtaining qualitative data by means of TLC chromographic analysis and quantitative data by phosporous determination. Gas chromographic analysis was made of the fatty acid content in liposomes separated by centrifuging from the liquid in which the excised lungs had been washed. No significant differences were observed as compared with the control group. Likewise, no significant differences were observed, as compared to the control group, regarding the amount of type II pneumocytes and their granular content (fixed and colored with Os-I).

5. Pharmacokinetics

The pharmacokinetic tests (titrimetric method on urine), after the oral administration of 200 mg/kg of the esters, in the rats, showed preferential renal excretion, within 24 hours after administration, to the extent of 60% of the original dose in an unaltered form and 40% in the form of non-sulfhydrylic, sulfurated organic compounds. When administering i.p., these percentages go up, respectively, to about 85% and 15%. On the other hand, the hematic peak is obtained within one hour, for oral administration, and within one-half hour for i.p. administration. Hematic levels for the new products are still determinable four hours after administration.

This represents a substantial difference from other thiols (derivatives of cysteine, cysteinamie, penicillamire) which, instead, are prevalently excreted as inorganic sulfates be-

BAD ORIGINAL

cause of their being completely metabolized.

These results demonstrate the ready absorbment and rapid excretion of the compounds through the urine. It is deduced, from these facts, that the new compounds unexpectedly act, prevalently, in this way. This characteristic probably constitutes the most surprizing and interesting aspect of the new products.

The new compounds can be administered orally, rectally or parenterally, diluted and supported by the usual, well-known pharmaceutical excipients.

Just by way of simple illustration, we herewith submit the two, typical, tested formulas shown below:

- Pills or capsules

| | |
|---|---|
| Thiolic ester | 750 mg |
| Ethylcellulose | 150 mg |

S. 4 pills per day

- Suppositories

| | |
|---|---|
| Thiolic ester | 300 mg |

Fatty acid triglycerides q.b. to 3 g

S. 3 per day

BAD ORIGINAL

Claims

1. The compounds having the formula :

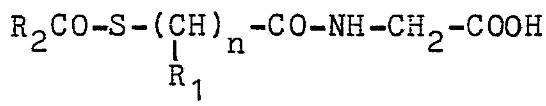 (I)

in which $R_1$ = H, $CH_3$; $R_2$ = alkylic radical, linear or with 8-20 carbon-atom branches, n = 1 or 2 and their salts.

2. Compounds, according to claim 1, having the formula:

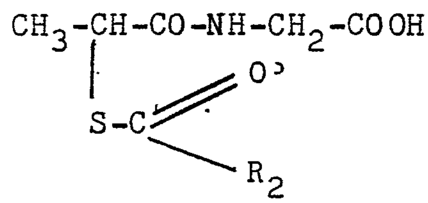

in which $R_2$ is as defined and their salts.

3. Compounds, according to claim 1, having the formula:

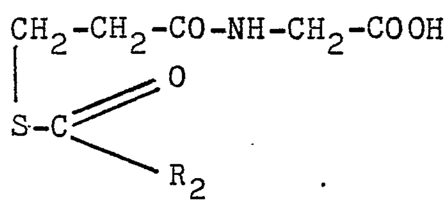

in which $R_2$ is as defined and their salts.

4. Process for the preparation of the compounds of the formula :

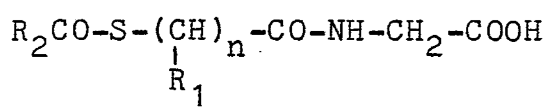

in which $R_1$ = H, $CH_3$; $R_2$ = alkylic radical, linear or with 8-20 carbon-atom branches; n = 1 or 2
characterized by the fact that bromo-propionamidoacetic acid is made to react with a thioacid according to the equation:

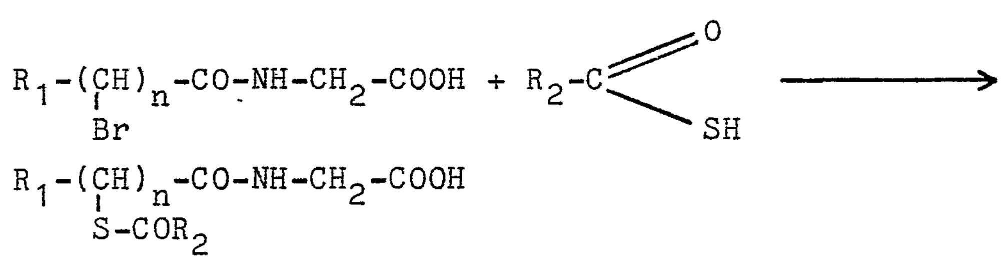

in which $R_1$, $R_2$ and n are as previously defined and the ob-

BAD ORIGINAL

0086757

CLAIMS (AUSTRIAN)

1. Process for the preparation of the compounds of the formula:

$$R_2CO-S-(\underset{R_1}{CH})_n-CO-NH-CH_2-COOH$$

in which $R_1$ = H, $CH_3$; $R_2$ = alkylic radical, linear or with 8-20 carbon - atom branches; n = 1 or 2 characterized by the fact that bromo-propionamidoacetic acid is made to react with a thioacid according to the equation:

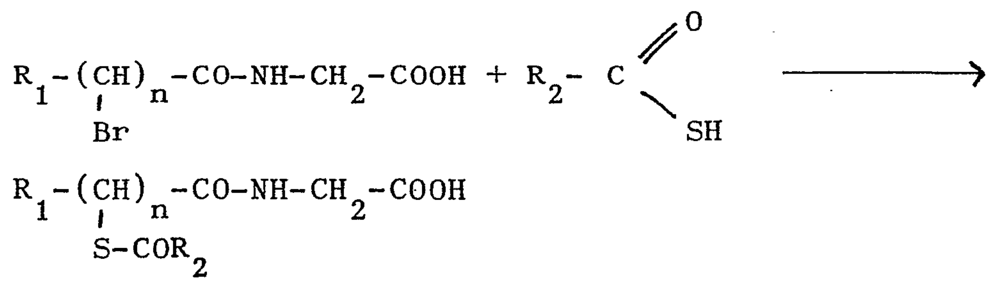

in which $R_1$, $R_2$ and n are as previsously defined and the obtained product is eventually salified.

2. Process, according to claim 1, in which the reaction is carried out in aprotonic, dipolar solvents, mixable with water, in the presence of an organic base and at room temperature.

BAD ORIGINAL

tained product is eventually salified.

5. Process, according to claim 4, in which the reaction is carried out in aprotonic, dipolar solvents, mixable with water, in the presence of an organic base and at room temperature.

6. Therapeutic compositions having mucolytic mucoregulating action, hepatotrophic-disintoxicating action, antihypertensive action and characterized by the fact they contain a compound having the following formula as active principle:

$$R_2CO-S-(\underset{\substack{| \\ R_1}}{CH})_n-CO-NH-CH_2-COOH$$

in which $R_1$, $R_2$ and n are as previously defined, mixed with diluents and inert excipients which are pharmacologically acceptable.

0086757

European Patent Office

# EUROPEAN SEARCH REPORT

Application number

EP 83 83 0030

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X | CHEMICAL ABSTRACTS, vol. 96, no. 18, 3rd May 1982, page 406, no. 148985c, Columbus, Ohio, USA & JP - A - 81 154 409 (POLA CHEMICAL INDUSTRIES INC.) 30-11-1981 * Whole abstract * --- | 1-5 | C 07 C 153/09<br>A 61 K 31/265//<br>C 07 C 103/48 |
| X | US-A-3 246 025 (ITARU MITA) * Example IX; column 2, lines 11-21 * ----- | 1-6 | |
| | | | **TECHNICAL FIELDS SEARCHED (Int. Cl. 3)** |
| | | | C 07 C 153/00<br>A 61 K 31/00 |

The present search report has been drawn up for all claims

| Place of search | Date of completion of the search | Examiner |
|---|---|---|
| THE HAGUE | 11-05-1983 | PAUWELS G.R.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82